# FASCICULE DE BREVET EUROPEEN

(11) **EP 2 255 196 B1**
(45) Date de publication et mention de la délivrance du brevet: **02.11.2011**
(21) Numéro de dépôt: 09718993.0
(22) Date de dépôt: 30.01.2009
(51) Int. Cl.: G01N 33/569, G01N 33/68

(54) **PROCEDE DE DETECTION ET/OU DE QUANTIFICATION ET/OU D'IDENTIFICATION IN VITRO DE COMPOSES INFECTIEUX DANS UN MATERIAU BIOLOGIQUE**
VERFAHREN FÜR IN VITRO NACHWEIS UND/ODER QUANTIFIZIERUNG UND/ODER IDENTIFIZIERUNG INFEKTIÖSER VERBINDUNGEN IN BIOLOGISCHEM MATERIAL
METHOD FOR IN VITRO DETECTION AND/OR QUANTIFICATION AND/OR IDENTIFICATION OF INFECTIOUS COMPOUNDS IN A BIOLOGICAL MATERIAL

(30) Priorité: 01.02.2008 FR 0800553
(43) Date de publication de la demande: 01.12.2010
(73) Titulaire: Apoh Technologies SA, 34280 La Grande Motte (FR)
(72) Inventeur: STEFAS, Ilias, F-34280 La Grande Motte (FR)
(74) Mandataire: Galup, Cédric Olivier Nicolas
(86) Numéro de dépôt international: PCT/FR2009/000104
(87) Numéro de publication internationale: WO 2009/112701

(56) Documents cités:
- EP-A- 0 775 315
- WO-A-94/18569
- FR-A- 2 723 203
- FR-A- 2 723 204

## Description

La présente invention concerne un procédé de détection et/ou de quantification et/ou d'identification in vitro de composés infectieux dans un matériau biologique

Dans la présente demande de brevet, on entend par "matériau biologique" un tissu biologique, une préparation ou un extrait issu de tissu biologique, liquide ou solide, ou un milieu, naturel ou non, susceptible de contenir des composés infectieux, par exemple une eau d'écoulement ou une eau de rinçage de fruits et légumes. Un tel matériau peut aussi être un mélange d'au moins deux matériaux tels que ci-dessus définis ; il peut donc être, notamment, soit préparé à partir de tissus, d'organes, de selles ou de liquides biologiques d'un malade atteint d'une infection, soit obtenu à partir de cultures "in vitro" ; un tel matériau biologique peut être aussi un sérum, du plasma, de l'urine, du liquide céphalo-rachidien, du liquide synovial, du liquide péritonéal, du liquide pleural, du liquide séminal ou du liquide acétique.

Dans la présente demande de brevet, on entend par composés infectieux, ci-après désignés de façon générique en abrégé par "CI", aussi bien des composés, en particulier protéiniques, constitutifs d'un agent infectieux, que des structures qui comprennent des composés infectieux. Ces structures sont, notamment, ou bien des agents infectieux endogènes ou exogènes, complets ou incomplets, leur métabolite ou encore des assemblages contenant des composés constitutifs de ces agents infectieux, assemblages qui présentent certaines propriétés desdits agents infectieux, notamment la propriété d'être détecté par certains anticorps spécifiques de composés infectieux ; les CI peuvent être aussi des composés spécifiquement induits dans l'organisme par les CI précédemment définis, ou par l'expression de gènes s'exprimant de façon anormale. Parmi les CI on peut citer, par exemple, les virus, les bactéries, les champignons, les mycoplasmes, les parasites et des cellules animales anormales.

On a déjà décrit une glycoprotéine plasmatique appelée β2-glycoprotéine I, ou encore en abrégé "β2GPI" ; la séquence de cette glycoprotéine humaine a notamment été indiquée dans les articles de J. LOZIER et coll., Proc. Natl. Acad. Sci. ISA, Vol. 81, p. 3640-3644 (juillet 1984) et de T. KRISTENSEN et coll., FEBS Letters, Vol. 289, p.183-186 (1991). Il a été constaté que cette protéine β2GPI présente un polymorphisme : la dénomination β2GPI sera considérée ci-après comme générique pour toutes les formes.

Dans la demande internationale WO 94/18569, on a indiqué que certains composés infectieux, en particulier protéiniques, se fixaient sur la forme de β2GPI qui avait été décrite dans le brevet français 2 701 263. On a proposé dans le document WO 94/18569, un procédé de détection et/ou de dosage de composés viraux dans lequel on fixe les composés infectieux viraux sur la forme de β2GPI utilisée ; on ajoute donc cette forme de β2GPI sur des composés infectieux viraux contenus dans un matériau biologique, de façon à séparer les composés viraux ainsi capturés pour ensuite les détecter et/ou les doser. Dans le brevet européen EP 775 315, on a décrit la formation d'un complexe entre un composé infectieux, en particulier protéinique, et une forme quelconque de β2GPI ; le composé infectieux pouvait, notamment, être une bactérie. Il ressort de ces documents que la β2GPI est susceptible de se fixer sur un support solide plan, tel que les fonds de puits d'une plaque de microtitration, et que la β2GPI ainsi accrochée sur ce support solide plan, est susceptible de fixer des composés infectieux (CI) présents dans des échantillons cliniques, biologiques ou environnementaux à de très faibles concentrations. On sait, en outre, que de tels échantillons peuvent contenir des substances inhibant, au moins partiellement, la détection des pathogènes, substances qui, par conséquent, peuvent diminuer la sensibilité de la détection. Il est donc important de pouvoir capturer et concentrer ces pathogènes pour éliminer les substances qui inhibent leur mise en évidence.

Les études de la société demanderesse ont montré que la fixation de la β2GPI sur le fond des puits des plaques de titration, se faisait grâce à une conformation particulière de la β2GPI, conformation qui permettait ultérieurement la formation d'un complexe de la β2GPI avec un composé infectieux. La littérature avait d'ailleurs signalé que la conformation de la β2GPI variait à sa fixation sur une surface solide (Matsuura et autres, J. Exp. Med. 179, p. 457-462 (1994). On avait déjà décrit (A. IWATA et autres, Biol. Pharm. Bull. 26(8), p. 1065-1069 (2003)) un procédé de concentration de virus utilisant des microbilles magnétiques sulfonées sur lesquelles les virus venaient s'accrocher, la concentration des virus étant obtenue grâce au fait que les microbilles étaient magnétiques et pouvaient être séparées du milieu infectieux par action d'un champ magnétique. Malheureusement le résultat de cette technique était essentiellement fonction de l'accrochage des virus sur les microbilles. Ce document explique de façon détaillée que certains virus non enveloppés ne se fixent pas sur des microbilles en polyéthylène-imine et qu'il est nécessaire d'utiliser des microbilles sulfonées pour concentrer certains virus. En outre, pour certains virus, il était nécessaire d'ajouter dans le milieu des cations bivalents, tels que Zn²⁺ ou Cu²⁺. Il résulte de cette constatation que, selon la nature du virus, le polymère constituant les microbilles doit être différent, greffé ou non, et que des ions bivalents sont nécessaires ou non ; les billes doivent donc être préparées au coup par coup en fonction du virus à concentrer. Les mêmes constatations résultent du document E. UCHIDA et autres, Journal of Virological Methods, 143, p. 95-103 (2007), qui est relatif à la concentration des virus des hépatites A, B, C humaines. En présence d'un échantillon contenant un virus non identifié à détecter, il n'était donc pas possible de déterminer quelle nature de microbilles était susceptible de donner lieu à un accrochage du virus d'intérêt.

En conséquence, compte tenu des inconvénients susmentionnés, un homme de métier aurait été enclin à rechercher des conditions environnementales favorables pour que les microbilles puissent accrocher des composés infectieux quelle que soit leur nature ou leur identité. C'est d'ailleurs la démarche suivie par UCHIDA dans son article précité ; il a décrit les conditions particulièrement favorables à la capture directe des virus HAV, HBV et HCV. La société demanderesse a, néanmoins, été à l'encontre de cette démarche en proposant, selon la présente invention, d'interposer, entre une microbille et un composé infectieux (CI) à fixer dessus, une molécule de β2GPI. L'état de la technique a permis de déterminer la nature des supports solides permettant un bon accrochage de la protéine β2GPI ; la fixation de la β2GPI sur une microbille s'effectue alors sans que le polymère de la microbille ait à être modifié en fonction du composé infectieux à fixer ultérieurement. Et, en outre, on a constaté que la fixation de la β2GPI sur la microbille ne perturbait pas l'accrochage du composé infectieux sur la β2GPI ; or, ce dernier point était totalement inattendu car on ne pouvait pas prévoir que la conformation de la β2GPI fixée sur une microbille, permettrait l'accrochage d'un agent pathogène sur la glycoprotéine. Au demeurant et à titre complémentaire, un élément dissuasif pour aboutir à l'invention provenait du fait que l'on savait que la β2GPI avait une tendance à s'auto-polymériser (voir : Thrombosis Research, 108, p. 175-180 (2003)), ce qui risquait d'entraîner une agglutination des microbilles porteuses de β2GPI, agglutination qui, bien entendu, rendait impensable la fixation d'agents pathogènes sur les molécules de β2GPI.

Enfin, on a constaté, selon l'invention, que l'accrochage des composés infectieux sur des microbilles chargées de β2GPI permettait, dans certains cas, par contact direct des microbilles chargées de β2GPI et de CI avec un milieu approprié, de détecter, quantifier ou identifier des CI accrochés. Dans le cas des virus, ledit milieu est une culture de cellules susceptibles d'être infectées par les virus capturés par les microbilles.

Selon un autre aspect de l'invention, la société demanderesse a aussi cherché, parallèlement, à assurer un bon accrochage des CI sur les microbilles chargées en β2GPI en agissant sur l'environnement où s'effectue cet accrochage ; elle a constaté que ce but était atteint lorsqu'on ajoutait dans le milieu d'accrochage des ions d'un métal oxydant ce, quelle que soit la nature des CI, notamment virus ou bactéries. Parmi ces métaux, figure le Cu²⁺ mais cet effet n'est pas dû au fait que le Cu est divalent (contrairement à ce que l'on pourrait penser à l'étude de la publication IWATA précitée) car IWATA mentionne qu'il obtient un résultat satisfaisant avec le Zn²⁺ ou le Cu²⁺ alors que la demanderesse a constaté que, pour la mise en oeuvre de l'invention, le Zn ne donne pas de résultats significatifs. On avait d'ailleurs déjà indiqué que la présence d'ions Zn ou Fe était défavorable pour l'accrochage des virus sur la β2GPI (STEFAS et autres, Hepatology, 33(1), pages 207-217 (2001)) ; or la présence d'éléments magnétiques dans les microbilles peut générer une diffusion défavorable d'ions fer dans le milieu de suspension des microbilles. La société demanderesse n'a constaté aucun effet notable défavorable à la fixation des CI sur la β2GPI quand on assure la présence d'ions oxydants dans le milieu.

La présente invention a, en conséquence, pour objet un procédé de détection et/ou de quantification et/ou d'identification in vitro de composés infectieux présents dans un milieu fluide M constituant un matériau biologique, procédé dans lequel, de façon connue, on prépare une suspension, dans un milieu liquide de suspension, de microbilles délimitées par une surface externe constituée d'un matériau polymère solide susceptible de fixer des protéines, caractérisé par le fait qu'il, comporte les étapes suivantes :
a) on assure un chargement des micro billes de la suspension avec des protéines β2GPI par couplage avec une quantité suffisante de protéines β2GPI, soit de façon passive dans un milieu de suspension, soit en utilisant un protocole de liaison chimique connu ;
b) on met en contact, dans un conteneur, lesdites microbilles chargées en protéines β2GPI avec le milieu fluide M en ajoutant des ions d'au moins un métal oxydant, dans des conditions appropriées pour assurer une fixation suffisante des composés infectieux sur les protéines β2GPI portées par les microbilles ;
c) on sépare les microbilles ainsi préparées de leur milieu de suspension, on évacue ledit milieu de suspension hors du conteneur pour obtenir éventuellement après un lavage par un tampon, un résidu à forte concentration de composés infectieux ;
d) et on détecte et/ou quantifie et/ou identifie les composés infectieux à partir du résidu concentré ainsi obtenu.

De préférence, les ions de métal oxydant qui, dans l'étape b) du procédé selon l'invention, sont ajoutés dans le milieu M, sont des ions cuivrique, la concentration d'ions cuivrique dans ledit milieu étant comprise, après ladite addition, entre 1 et 100 mM.

Avantageusement, on réalise l'étape d) du procédé selon l'invention ci-dessus défini par un moyen pris dans le groupe formé par l'infectiosité, une réaction enzymatique spécifique, un traceur fluorescent ou radiomarqué, une détection d'acides nucléiques spécifiques par hybridation avec une sonde marquée, une réaction PCR ou RT-PCR, un dosage, une numération, une visualisation, un procédé optique, une microscopie électronique ou non.

Les composés infectieux (CI) peuvent, notamment, être des bactéries.

Dans le cas où les CI sont des bactéries, on peut les détecter et/ou quantifie et/ou identifier par lecture de densité optique, par ATP-métrie ou par PCR (« polymerase chain réaction » ou «réaction en chaine obtenue avec une polymérase »).

Dans le cas où les CI sont des virus, on extrait par lyse, à partir du résidu concentré obtenu à la fin de l'étape c) du procédé, les acides nucléiques des CI d'intérêt, on amplifie par PCR (ou RT-PCR si les virus d'intérêt sont des rétrovirus) les acides nucléiques des CI d'intérêt en utilisant les amorces appropriées auxdits CI d'intérêt et on pratique une visualisation sur gel des acides nucléiques éventuellement obtenus pour définir la présence ou l'absence des CI d'intérêt et/ou pour quantifier la charge desdits CI d'intérêt dans le milieu M. Avantageusement, pour détecter et/ou identifier des virus d'intérêt fixés sur des microbilles obtenues selon l'étape c) du procédé selon l'invention, on re-suspend le résidu, on met en contact ses microbilles avec des cellules sensibles au virus d'intérêt, on met en culture lesdites cellules et on observe l'éventuelle infection des cellules par les virus d'intérêt. Pour détecter une infection de cellules, on peut, par exemple, observer l'effet cytopathologique du composé infectieux après une coloration appropriée des cellules ou encore observer l'immunofluorescence après fixation des cellules et réaction avec des anticorps fluorescentes, qui reconnaissent des protéines correspondant à la présence des composés infectieux.

On choisit, de préférence, le matériau solide constitutif de la surface externe des microbilles dans le groupe formé par les matières plastiques et les élastomères, ledit matériau portant ou non des groupements réactifs greffés sur la surface externe des microbilles pour assurer une liaison chimique avec les protéines β2GPI ; les microbilles peuvent avantageusement avoir une forme sensiblement sphérique et un diamètre moyen compris entre 1 et 100 000 nm. Selon une première variante, on sépare les microbilles de leur milieu de suspension par centrifugation ; mais selon une deuxième variante préférée, on choisit des microbilles ayant un coeur formé d'une (ou de) particule(s) de matériau magnétique pour permettre leur séparation par rapport au milieu de suspension grâce à un champ magnétique. De telles microbilles magnétiques sont disponibles dans le commerce : par exemple, elles sont constituées d'un coeur magnétique recouvert d'une matrice polymérique de polystyrène. Le champ magnétique permettant la séparation des microbilles par rapport à leur milieu de suspension, peut être créé par un simple aimant permanent que l'on approche du conteneur pour réaliser l'étape c) du procédé selon l'invention.

La seule limite concernant le choix du matériau constitutif des microbilles, est de pouvoir coupler la β2GPI : on peut, par exemple, utiliser des microbilles magnétiques correspondant à la dénomination commerciale "Estapor® microsphères superparamagnétiques" vendues par la société "MERCK". Comme précédemment indiqué, le couplage de la β2GPI sur les microbilles peut se faire soit de façon passive, soit en utilisant un protocole de couplage chimique. Pour réaliser un couplage passif avec des microbilles, notamment les microbilles "Estapor"® précitées, on met, avantageusement, les microbilles en suspension dans un tampon contenant de la β2GPI, à un pH compris entre 3,5 et 10,5 et mieux, entre 5,5 et 9,5. Le tampon utilisé fait partie des tampons courants en biologie et, notamment, peut être un tampon acétate, phosphate, borate, Tris. Le mélange microbille/β2GPI est, de préférence, incubé à une température comprise entre 4°C et 40°C pendant un temps compris entre 10 mn et 24 h sous agitation, de préférence une agitation horizontale douce et constante. Par la suite, les microbilles sont séparées magnétiquement ou centrifugées et le surnageant est éliminé. Le culot contenant les microbilles est mis en suspension dans un tampon de conservation, qui est, de préférence, le même que celui utilisé ultérieurement pour le couplage, ce tampon ayant un pH compris entre 6 et 9. De préférence, on effectue le chargement des microbilles en protéines β2GPI en les mettant dans un milieu liquide de suspension qui contient, en solution aqueuse, de 10⁻⁶ à 100 mg de β2GPI par gramme de poids sec de microbilles, la concentration en β2GPI du milieu étant alors comprise entre 10⁻⁵ et 10 µg/µl, et en agitant la suspension ainsi constituée pendant 15 à 60 mn à une température comprise entre 30°C et 45°C.

L'échantillon contenant le CI est mis en contact avec les microbilles chargées, soit directement, soit après sa dilution dans un tampon, dont le pH est compris entre 5 et 9, mieux entre 5,6 et 8. Le complexe, qui se forme entre la β2GPI et le CI, est, avantageusement, par la suite, incubé pendant une période de temps comprise entre 5 mn et 24 h, de préférence, entre 30 mn et 2 h, à une température comprise entre 4°C et 40°C, de préférence environ 37°C. Après incubation, l'échantillon qui n'a pas réagi avec la β2GPI fixée sur les microbilles, est éliminé par centrifugation ou aimantation des microbilles. Les microbilles ainsi isolées peuvent être utilisées pour la détection et/ou la quantification et/ou l'identification du CI. La séparation et/ou le dosage et/ou la quantification du CI lié au support par la β2GPI peut se faire par tout moyen connu tel que l'infectiosité, une réaction enzymatique spécifique, un traceur fluorescent ou radiomarqué, la détection d'acide nucléique spécifique par hybridation avec une sonde marquée, une réaction en chaîne obtenue avec une polymérase, un dosage, une numération, une visualisation, un procédé optique, une microscopie électronique ou non.

Pour mieux faire comprendre l'objet de l'invention, on va en décrire maintenant, à titre d'exemples purement illustratifs et non limitatifs, plusieurs modes de mise en oeuvre.

### EXEMPLE 1 : Fixation d'une bactérie en milieu cuivrique sur des microbilles chargées en β2GPI

Dans un premier temps, la bactérie que l'on a utilisée, est une souche d'Escherichia Coli (E. Coli) fournie par le Centre de conservation de produits agricoles (CPA). Une pré-culture est incubée à 37°C pendant 16 h dans du milieu LB (Luria Bertani) ayant la composition suivante :

| | |
|---|---|
| Bacto tryptone | 10 g |
| Extrait de levure | 5 g |
| NaCl | 10 g |
| pH | 7,5 |
| Eau | qsp 1 000 g |

Cette pré-culture est utilisée immédiatement ou conservée à 4,5°C.

Les microbilles destinées à fixer les bactéries que l'on utilise dans cet exemple sont des microbilles magnétiques vendues par la Société MERCK sous la dénomination "Estapor® microsphères superparamagnétiques" qui ont un diamètre compris entre 0,300 et 0,500 µm.

Ces microbilles sont mises en suspension dans un tampon à un pH de 6,0 contenant la β2GPI. La concentration de β2GPI dans ce tampon de couplage est de 100 µg/ml ; les microbilles sont incubées dans le tampon sous agitation douce et constante à une température de 25°C pendant 3 heures. Les microbilles sont ensuite centrifugées à 1 500 tours/minute et le surnageant est éliminé ; le culot de centrifugation est mis en suspension dans le même tampon que celui utilisé pour le couplage ultérieur de la β2GPI, ce qui forme la suspension de microbilles chargées en β2GPI que l'on veut tester.

10⁵ bactéries de la préculture sont mises en suspension soit dans du tampon Tris 50 mM (pH 7,6), soit dans du tampon PBS (voir formulation ci-après), soit dans un tampon acétate de sodium 50 mM (pH 5,6 avec HCl 10 mM), en présence ou absence de 2 mM de CuSO4, soit dans un milieu LB ; les différentes suspensions de E. Coli sont placées dans des tubes à hémolyse de 1 ml avec une quantité de microbilles constante (10 µl). Les tubes sont mis incubés à 37°C pendant 60 mn, sous agitation horizontale. Dans chaque tube, on sépare ensuite les microbilles de la phase liquide au moyen d'un aimant permanent placé extérieurement contre la paroi du tube et on évacue le surnageant. Les microbilles sont ensuite lavées deux fois avec du PBS stérile ayant la formulation suivante :

| | |
|---|---|
| NaCl | 80 g |
| KCl | 74,562 g |
| KH₂PO₂ | 2,4 g |
| Na₂HPO₄/2H₂O | 29 g |
| Eau | qsp 1 000 g |

La présence de bactéries est évaluée par PCR. Le tableau I résume les résultats obtenus :

**Tableau I**

| Tampon | PCR |
|---|---|
| Tris | + |
| PBS | + |
| Acétate Na | + |
| Acétate Na/Cu²⁺ | ++ |
| LB | + |

L'ADN bactérien est extrait à partir des bactéries qui ont été capturées par les microbilles ; les bactéries sont lysées en ajoutant aux microbilles 100 µl de "Chelex 30 %". Le mélange est incubé pendant 10 minutes à 95°C ; on effectue ensuite une centrifugation pendant 10 minutes à 10 000 tours/minute. Le surnageant contenant l'ADN, est conservé à -20°C.

A 3 µl de l'ADN extrait sont ajoutés 47 µl de la solution d'amplification (AquaPure Génomic DNA Isolation KIT) ; les concentrations finales sont les suivantes :
- 5 µl : dXTP 200 mM
- 10 µl : Tampon 5X
- 5 µl : MgC12 2mM
- 1 µl de chaque amorce : (amorce diluée à 200 mL) :
   27 : GTGCTGCAGAGAGTTTGATCCTGGCTCAG (sens)
   1492 : CACGGATCCTACGGGTACCTTGTTACGACTT (antisens)
- 1 µl : Taq polymerase 5u/µL
- Eau PPI qsp 50µL

Après homogénéisation, les mélanges réactionnels sont placés dans un thermocycleur "Eppendorf" et soumis au programme suivant :

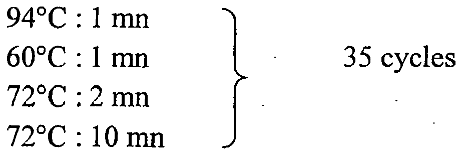

Les ADN sont ensuite maintenus à 10°C. La migration se fait sur un gel d'agarose à 2 % en tampon PBE 0,5X contenant du bromure d'éthydium. Le gel est alors observé sous lumière UV. Les résultats de la PCR indiquent bien la présence d'une bactérie : on constate un fort signal positif. L'identification de la bactérie se fait ensuite par séquençage de façon connue. La PCR montre la présence d'ADN bactérien sur les microbilles quelque soit le tampon utilisé avec un signal supérieur pour le cas du tampon acétate en présence de Cu²⁺

Dans un deuxième temps, des essais analogues ont également été menés avec les bactéries *Pseudomonas aeruginosa, Streptococcus pneumoniae et Staphylococcus aureus* et ont donné le même type de résultats.

### EXEMPLE 2 : Fixation en milieu cuivrique du virus de l'herpès OsHV-1 sur des microbilles chargées en β2GPI

Ce virus existe dans les huîtres. On réalise un broyat d'huîtres dont on extrait l'ADN par PCR au moyen de microbilles magnétiques chargées en β2GPI. Ces microbilles chargées sont préparées comme indiqué à l'exemple 1.

100 mg de jeunes huîtres sont broyés dans 500 µl d'eau de qualité milli-Q. Le broyat (50 µl) a été dilué dans deux tampons différents à savoir, un tampon PBS (pH = 7-7,4) (composition identique à celle de l'exemple 1) et un tampon Acétate/Cu²⁺ (pH = 5,6) ayant la composition définie à l'exemple 1.

On a ajouté à 500 µL de ce qui constitue ainsi deux milieux fluides M, des quantités de microbilles chargées de 10, 20 ou 50 µL. Les billes sont ensuite incubées à 37°C pendant 30 mn et lavées 2 fois avec du tampon PBS. L'ADN est enfin extrait en ajoutant 100 µl de « Chelex 30% ». On a laissé incubé pendant 10mn à 95°C. On fait subir à tous les tubes une centrifugation à 10000 tours/mn pendant 10 mn et l'on conserve le surnageant qui contient l'ADN.

On pratique ensuite une PCR sur 1µL de l'ADN extrait. On ajoute 19 µL d'une solution d'amplification (kit PCR Hot Master Taq Eppendorf) aux concentrations finales suivantes :

| | |
|---|---|
| Tapon | 1X |
| dNTP | 250 µM |
| Amorces C2 et C6 | 0,2 µM chacune |
| Taq Polymérase | 1,5 U |

Les amorces C2 et C6 sont les suivantes :
C2 (sens) = CTCTTTACCATGAAGATACCCACC
C6 (antisens) = GTGCACGGCTTACCATTTTT
Le mélange réactionnel est homogénéisé et placé dans un thermocycleur « Master cycler personal Eppendorf » et soumis au programme suivant :

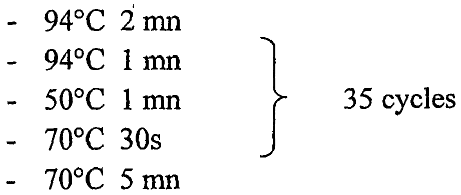

L'ADN est ensuite conservé à 10°C. On a également effectué le même travail en remplaçant le broyat d'huîtres par 5, 10 puis 20 mL d'eau de mer (provenant du parc à huîtres), la quantité de microbilles chargées de β2GPI étant alors constante et égale à 50 µL.

A titre de comparaison, on a soumis le broyat d'huîtres à une extraction directe des ADN de virus par les deux produits de lyse ci-dessus définis, sans addition préalable de microbilles chargées et on a utilisé les mêmes conditions de PCR.

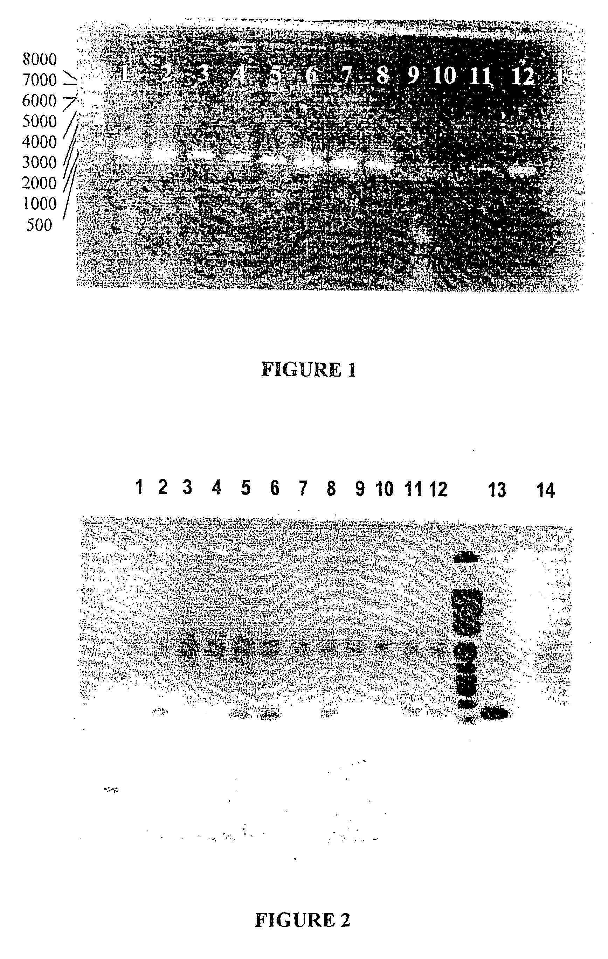

Dans tous les cas, les ADN obtenus en PCR ont été utilisés pour faire un Souhem Blot sur gel d'agarose à 2 % en tampon PBE 0,5X contenant du bromure d'éthydium. Le gel est alors observé sous lumière UV. L'ensemble des résultats est présenté sur la figure 1 sur laquelle les pistes 1 à 13 représentent les expériences définies par le tableau II :

**Tableau II**

| Piste de la figure 1 | Expérience |
|---|---|
| 1 | Broyat + PBS + 10µl microbilles |
| 2 | Broyat + PBS + 20µl microbilles |
| 3 | Broyat + PBS + 50µl microbilles |
| 4 | Broyat + Acétate Na/Cu2⁺ + 10µl microbilles |
| 5 | Broyat + Acétate Na/Cu²⁺ + 20µl microbilles |
| 6 | Broyat + Acétate Na/Cu²⁺ + 50µl microbilles |
| 7 | Broyat + extraction directe Chelex |
| 8 | Broyat + extraction directe phénol/chloroforme |
| 9 | Eau de mer 5 mL /50 µL microbilles |
| 10 | Eau de mer 10 mL / 50 µL microbilles |
| 11 | Eau de mer 20 mL / 50 µL microbilles |
| 12 | T⁺ |
| 13 | Tmix |

La figure 1 montre un même signal positif sur les pistes 1 à 8 : l'ADN du virus obtenu par extraction directe (pistes 7 et 8) est donc bien celui obtenu grâce aux microbilles (pistes 1 à 6). On a séquencé tous ces ADN : l'amplicon obtenu par les microbilles correspond à une séquence de 709 bases du gène de la glycoprotéine 1005 du virus OsHV-1.Lorsque le broyat est dilué dans du PBS (pistes 1 à 3), le signal est le même quelque soit la quantité de microbilles utilisée alors qu'en présence des ions Cu²⁺ (pistes 4 à 6), le signal est d'autant plus fort que la quantité de billes est plus grande ; en outre, en présence de Cu²⁺ (pistes 4 à 6), le signal est toujours plus fort qu'en absence de Cu²⁺ (pistes 1 à 3). Ceci démontre l'intérêt de la présence des ions Cu²⁺ qui améliorent la sensibilité de détection.

Sur les pistes 9 à 11, on voit qu'un signal apparaît faiblement pour la piste 11, alors qu'une extraction directe sur l'eau de mer avait donné un résultat négatif, ce qui montre également que, sans les ions Cu²⁺, la détection par les microbilles est faible

La piste T⁺ est un témoin positif obtenu avec la séquence d'ADN viral pour établir que la PCR a bien fonctionné ; la piste Tmix est un témoin obtenu avec tous les ingrédients mis en oeuvre dans la PCR mais en l'absence de l'ADN viral.

### EXEMPLE 3 : Détection du virus de l'hépatite C (VHC)

50 µL de sérum provenant de malades infectés par le virus de l'hépatite C sont dilués dans 500 µL de tampon acétate de sodium 50 mM (pH = 5,6 avec 10 mM d'HCl). On ajoute à ce milieu 10 µL de microbilles chargées en β2GPI, identiques à celles qui ont été préparées à l'exemple 1. On ajoute également des sels ferriques, cuivriques, de zinc et de manganèse de façon à obtenir un milieu dans lequel les ions métalliques sont présents à 2 mM. On laisse incuber cet ensemble à 37°C pendant 30 mn, sous agitation rotative. On amène ensuite, le long du tube qui contient l'échantillon, un aimant permanent, qui permet de séparer les microbilles de leur milieu de suspension, et on élimine le surnageant. On soumet les microbilles à une lyse selon de protocole du kit « QIAamp Viral RNA mini kit (Qiagen) ». Des acides nucléiques viraux sont soumis à un protocole de RT-PCR : Le protocole de RT-PCR a été antérieurement décrit par Young et al. (J. Clin. Microbiol., 1993, 31(4), p. 882-6). A 4 µL d'ARN extraits, sont ajoutés 21 µL de la solution d'amplification (kit RT-PCR One step Qiagen) aux concentrations finales suivantes :

| | |
|---|---|
| Qiagen tampon | 1X |
| dNTP | 400 µM |
| Amorces | 0,6 µM chacune |
| Taq Polymérase | 1,5 U |
| Inhibiteur de RNase | 15 U |

Les amorces utilisées sont les suivantes
KY78 (sens) : CAAGCACCCTATCAGGCAGT
KY80 (antisens) : AGCGTCTAGCCATGGCGT

Après homogénéisation les mélanges réactionnels sont placés dans un thermocycleur (Master cycler personnal Eppendorf) et soumis au programme suivant :

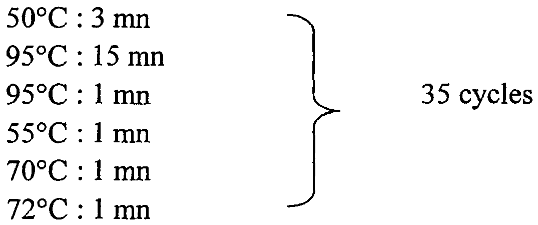

Les ADN sont ensuite maintenus à 4°C et l'on effectue un Southern Blot correspondant à la figure 2.

La figure 2 montre qu'en présence de Cu²⁺, la sensibilité de détection du virus de l'hépatite C est nettement améliorée. Au contraire, en présence de Zn²⁺, le virus n'est pas détectable, et ce, quelque soit le pH de l'échantillon.

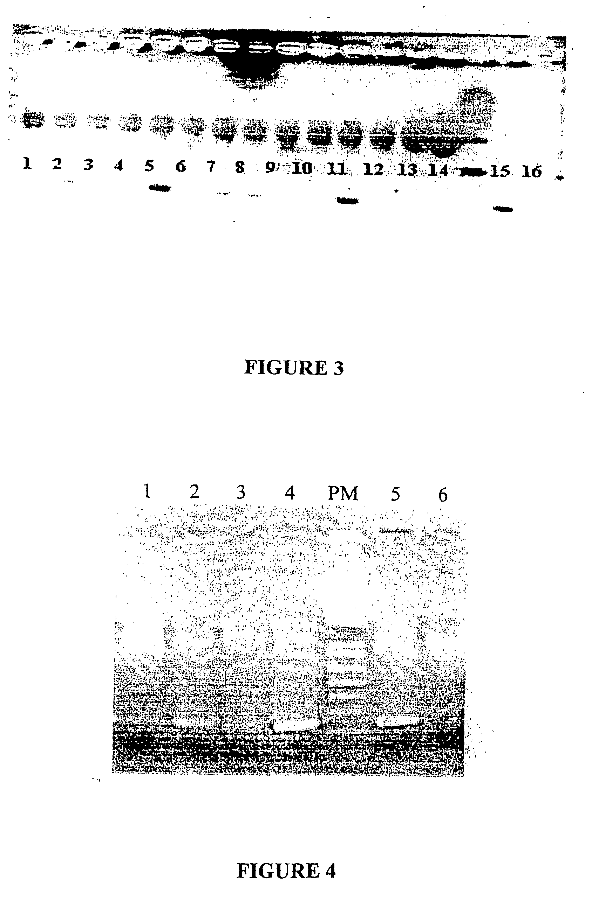

La figure 3 montre les résultats obtenus pour un échantillon (sérum ou plasma) contenant 130 copies de génome viral par mL d'échantillon. La définition des échantillons correspondant aux 16 pistes est donnée dans le tableau III. On constate sur la figure 3 qu'en l'absence de microbilles magnétiques chargées de β2GPI, il n'y a aucune signal montrant la présence virale : on a atteint la limite de sensibilité de la méthode utilisée. En revanche, en présence de Cu²⁺, le signal du virus devient visible.

**Tableau III**

| Piste n° | Echantillon testé | Piste n° | Echantillon testé |
|---|---|---|---|
| 1 | sérum pH 7,6 + 6 microbilles | 10 | plasma pH 5,6 + microbilles |
| 2 | sérum pH 7,6 + Cu⁺⁺+ microbilles | 11 | plasma pH 5,6 Cu⁺⁺ + microbilles |
| 3 | sérum pH 7,6 + Fe⁺⁺ + microbilles | 12 | plasma pH 5,6 Fe⁺⁺ + microbilles |
| 4 | sérum pH 5,6 + microbilles | 13 | sérum détection en absence de microbilles |
| 5 | sérum pH 5,6 + Cu⁺⁺ + microbilles | 14 | plasma détection en absence de microbilles |
| 6 | sérum pH 5,6 + Fe⁺⁺+ microbilles | 15 | contrôle positif |
| 7 | plasma pH 7,6 + microbilles. | 16 | contrôle négatif |
| 8 | plasma pH 7,6 + Cu⁺⁺ + microbilles | | |
| 9 | plasma pH 7.6 + Fe⁺⁺ + microbilles | | |

On a par ailleurs établi que les ions Cu²⁺ agissent sur l'échantillon et non sur les microbilles. Pour ce faire, on a ajouté de l'acétate cuivrique 2 mM sur des microbilles chargées en β2GPI. On a laissé incubé à 37°C pendant 30 mn sous agitation. Les microbilles ont été ensuite rincées et mises en contact avec l'échantillon de sérum ne contenant pas de Cu²⁺. Le tableau IV identifie la nature de l'essai correspondant à chacune des pistes 1 à 6 de la figure 4.

**Tableau IV**

| Piste n° | Nature de l'essai |
|---|---|
| 1 | β2GPI + Cu⁺⁺ + lavage + VHC |
| 2 | β2GPI + Cu⁺⁺ + VHC |
| 3 | VHC en absence de β2GPI |
| 4, 5 | Contrôle positif |
| 6 | Contrôle négatifI |
| PM | Poids moléculaire |

### EXEMPLES 4 A 8 : Détection d'autres virus

On a utilisé des protocoles d'essai identiques à ceux qui ont été décrits ci-dessus dans les exemples 2 ou 3 selon qu'il s'agit respectivement de virus à ADN ou à ARN.

On a constaté que pour tous ces virus, les résultats étaient les mêmes que ceux décrits dans les exemples 2 ou 3. Les virus qui ont fait l'objet de ces essais sont : les virus du West nile, le virus Antavirus type Andes, le virus de la Dengue sous-type 1, 2, 3 et 4, le virus HIV 1 et 2 et le virus influenza H1N1 et H1N2.

### EXEMPLE 9 Capture des virus Influenza H3N2 par des microbilles en milieu cuivrique et utilisation des microbilles chargées de virus pour la détection visuelles desdits virus.

Un prélèvement pharyngien d'un patient atteint d'un état grippal, est dilué dans du milieu de culture MEM (milieu essentiel minimum de « Eagle ») contenant 2 mM de sulfate cuivrique. On y ajoute 10 µL de microbilles telles que préparées à l'exemple 1 : ces microbilles sont donc chargées de β2GP1. Les microbilles sont mises en contact avec 500 µL du milieu où se trouve le prélèvement, le tout étant placé dans un tube Eppendorf de 2 mL. Le mélange est incubé à 37°C sous agitation rotative pendant 30 mn. Le tube est ensuite placé au contact d'un aimant permanent ; les microbilles sont attirées par le champ magnétique contre la paroi ; le surnageant est aspiré et éliminé. On ajoute dans le tube, 1,5 mL de tampon PBS (composition donnée à l'exemple 1) pour obtenir une suspension des microbilles ; le tube est replacé en contact avec l'aimant permanent et le surnageant est aspiré et éliminé. Cette opération de lavage des microbilles est répétée trois fois au total, puis les microbilles sont resuspendues dans 1 mL de tampon de culture MEM.

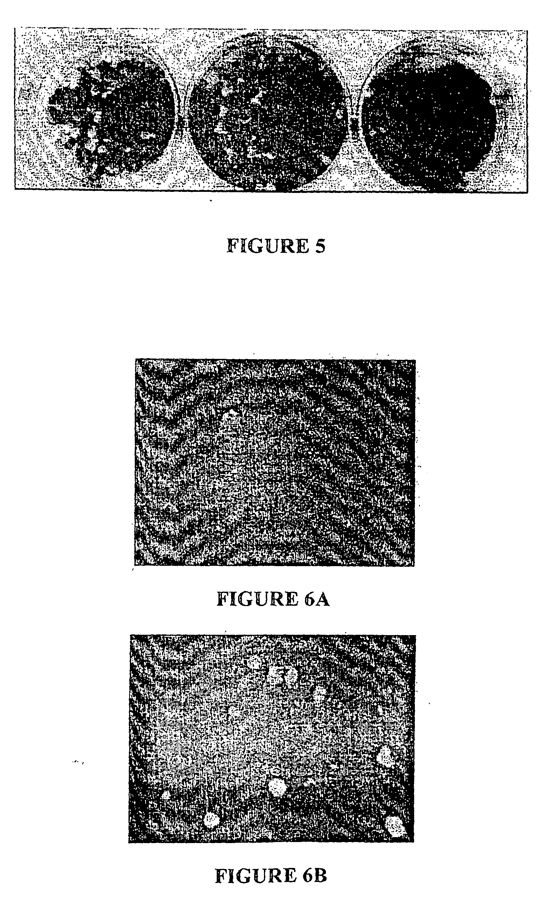

La suspension ainsi obtenue est mise en contact avec des cellules MDCK à 37°C pendant 24 heures. Les cellules sont ensuite lavées deux fois en sérum physiologique et on ajoute du milieu de culture MEM frais et les cellules sont cultivées pendant 4 jours à 37°C. L'infection est vérifiée soit par l'effet cytopathogène du virus après coloration des cellules par le cristal violet (voir figure 5), soit par immunofluorescence, après fixation des cellules par l'acétone et réaction avec des anticorps monoclonaux fluorescents, qui reconnaissent les protéines virales (figures 6A et 6B).

Sur la figure 5, la boîte de Pétri à gauche, correspond à 6000 pfu de virus (1 pfu permet de former un foyer de lyse), la vue du milieu correspond à 2000 pfu de virus et la vue de droite correspond à 200 pfu de virus. La disparition progressive des cellules montre que les microbilles portaient bien des virus à effet cytopathogène.

Sur la figure 6A, on voit que les cellules portant des protéines virales sont repérées par fluorescence : on a mis le milieu de culture contenant le prélèvement viral directement en contact avec les cellules MDCK et on constate que peu de cellules sont fluorescentes ce qui montre que la capture du virus a été de faible importance en l'absence d'utilisation des microbilles. La figure 6B montre le résultat obtenu avec l'utilisation des microbilles : on établit ainsi que les microbilles ont concentré les virus du prélèvement, ce qui permet donc une détection avec une charge virale beaucoup plus faible.

### EXEMPLE 10: Capture du virus de la vaccine par les microbilles chargées en β2GP1 et utilisation de ces dernières pour infecter des cellules.

Le même protocole que celui détaillé à l'exemple 9 est appliqué pour l'infection des cellules Hep 2, par le virus de la vaccine capturé par les microbilles chargées de β2GP1. Pour cet exemple, on a utilisé une suspension de virus permettant de former 1000 foyers de lyse (1000 pfu).

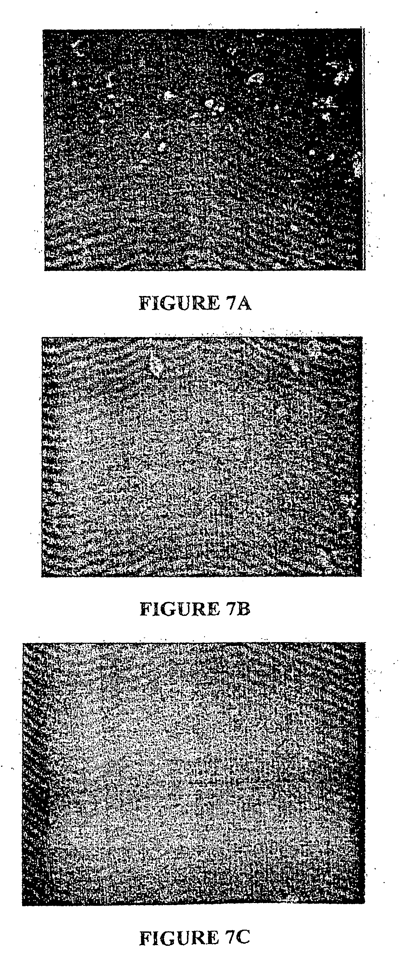

La figure 7A montre la situation lorsque le virus de la vaccine a été capturé par des microbilles chargées en β2GP1 : on voit que de nombreuses cellules ont été infectées. Au contraire, la figure 7B montre le cas où les cellules Hep 2 ont été infectées par le virus de la vaccine en l'absence de microbilles chargées de β2GP1 : on voit que le nombre de cellules fluorescentes est beaucoup plus restreint, ce qui permet de conclure que les microbilles chargées de β2GP1 ont permis de concentrer les virus utilisés, puisque la quantité de virus était la même pour les essais des figures 7A et 7B.

La figure 7C montre des cellules Hep 2 mises en contact avec le tampon de lavage des microbilles après qu'elles aient été chargées de β32GP1 et de virus de la vaccine : on constate qu'il y a absence de fluorescente, ce qui veut dire que le tampon de lavage n'a emmené aucun des virus fixés sur les microbilles.

On constate donc que les microbilles chargées en β2GP1 permettent d'améliorer la sensibilité de la détection des virus.

## Revendications

1. Procédé de détection et/ou de quantification et/ou d'identification in vitro de composés infectieux (CI) présents dans un milieu fluide M constituant un matériau biologique, procédé dans lequel, de façon connue, on prépare une suspension, dans un milieu liquide de suspension, de microbilles délimitées par une surface externe constituée d'un matériau polymère solide susceptible de fixer des protéines, **caractérisé par le fait qu'**il comporte les étapes suivantes :
a) on assure un chargement des microbilles de la suspension avec des protéines β2GPI par couplage avec une quantité suffisante de protéines β2GPI, soit de façon passive dans un milieu de suspension, soit en utilisant un protocole de liaison chimique connu ;
b) on met en contact, dans un conteneur, lesdites microbilles chargées en protéines β2GPI avec le milieu fluide M en ajoutant des ions d'au moins un métal oxydant, dans des conditions appropriées pour assurer une fixation suffisante des composés infectieux sur les protéines β2GPI portées par les microbilles ;
c) on sépare les microbilles ainsi préparées de leur milieu de suspension, on évacue ledit milieu de suspension hors du conteneur pour obtenir, éventuellement après un lavage par un tampon, un résidu à forte concentration de composés infectieux ;
d) et on détecte et/ou quantifie et/ou identifie les composés infectieux à partir du résidu concentré ainsi obtenu.

2. Procédé selon la revendication 1, **caractérisé par le fait que** l'on effectue le chargement des microbilles, dans l'étape a) de la revendication 1, de façon passive dans un tampon ayant un pH compris entre 3,5 et 10,5, en laissant incuber, pendant un temps compris entre 10 minutes et 24 heures, à une température comprise entre 4 et 40°C.

3. Procédé selon l'une des revendications 1 ou 2, **caractérisé par le fait que** l'on effectue le chargement des microbilles en protéines β2GPI en les mettant dans un milieu liquide de suspension qui contient, en solution aqueuse, de 10⁻⁶ à 100 mg de β2GPI par gramme de poids sec de microbilles, la concentration du milieu en β2GPI étant comprise entre 10⁻⁵ et 10 µg/µl.

4. Procédé selon l'une des revendications 1 à 3, **caractérisé par le fait que** le chargement des microbilles en protéines β2GP1 s'effectue dans un tampon ayant un pH compris entre 5 et 9 avec une incubation pendant un temps compris entre 10 minutes et 24 heures à une température comprise entre 4 et 40°C.

5. Procédé selon l'une des revendications 1 à 4, **caractérisé par le fait que** dans l'étape b), les ions de métal oxydant ajoutés dans le milieu M sont des ions cuivrique, la concentration d'ions cuivrique dans le milieu M étant comprise, après ladite addition, entre 1 mM et 100 mM.

6. Procédé selon l'une des revendications 1 à 5, **caractérisé par le fait que** l'on choisit le matériau solide constitutif de la surface externe des microbilles dans le groupe formé par les matières plastiques et les élastomères, ledit matériau portant ou non des groupements réactifs greffés sur la surface externe des microbilles pour assurer une liaison chimique avec les protéines β2GPI.

7. Procédé selon l'une des revendications 1 à 6, **caractérisé par le fait que** l'on choisit les microbilles ayant une forme sensiblement sphérique et un diamètre moyen compris entre 1 et 100 000 nm.

8. Procédé selon l'une des revendications 1 à 7, **caractérisé par le fait que** l'on choisit des microbilles ayant un coeur formé d'une (ou de) particule(s) de matériau magnétique pour permettre leur séparation par rapport au milieu de suspension grâce à un champ magnétique.

9. Procédé selon l'une des revendications 1 à 7, **caractérisé par le fait que** l'on sépare les microbilles de leur milieu de suspension par centrifugation.

10. Procédé selon l'une des revendications 1 à 9, **caractérisé par le fait que** l'on réalise l'étape d) de la revendication 1 par un moyen pris dans le groupe formé par l'infectiosité, une réaction enzymatique spécifique, un traceur fluorescent ou radiomarqué, une détection d'acides nucléiques spécifiques par hybridation avec une sonde marquée, une réaction PCR ou RT-PCR, un dosage, une numération, une visualisation, un procédé optique, une microscopie électronique ou non.

11. Procédé selon l'une des revendications 1 à 10, **caractérisé par le fait que** les composés infectieux sont des bactéries.

12. Procédé selon l'une des revendications 1 à 10, **caractérisé par le fait que** les composés infectieux sont des virus.

13. Procédé selon la revendication 11, **caractérisé par le fait que** l'on détecte et/ou quantifie et/ou identifie les bactéries du milieu fluide M à partir du résidu par lecture de densité optique, par ATP-métrie ou par PCR.

14. Procédé selon l'une des revendications 11 à 13, **caractérisé par le fait que** l'on extrait par lyse, à partir du résidu concentré, les acides nucléiques des CI d'intérêt, que l'on amplifie par PCR ou RT-PCR, lesdits acides nucléiques en utilisant les amorces appropriées auxdits CI d'intérêt et que l'on pratique une visualisation sur gel des acides nucléiques éventuellement obtenus pour définir la présence ou l'absence desdits CI d'intérêt et/ou pour quantifier la charge virale desdits CI d'intérêt dans le milieu M.

15. Procédé selon la revendication 12, **caractérisé par le fait que** pour détecter et/ou identifier des virus d'intérêt fixés sur des microbilles obtenues selon l'étape c) de la revendication 1, on resuspend le résidu, on met en contact ces microbilles avec des cellules sensibles aux virus d'intérêt, on met en culture lesdites cellules et on observe l'éventuelle infection des cellules par les virus d'intérêt.

16. Procédé selon la revendication 15, **caractérisé par le fait que**, pour détecter une infection de cellules, on observe l'effet cytopatholgique des virus après une coloration appropriée des cellules, ou l'immunofluorescence après fixation des cellules et réaction avec des anticorps fluorescents qui reconnaissent des protéines correspondant à la présence des virus.

## Claims

1. *In vitro* method for detection and/or quantification and/or identification of infectious compounds (ICs) present in a fluid medium M constituting a biological material, a method in which, in a known manner, a suspension of microbeads in a liquid suspension medium is prepared, said microbeads being delimited by an outer surface constituted by a solid polymer material capable of binding proteins, **characterized by** the fact that it comprises the following steps:
a) a loading of the microbeads in the suspension with β2GPI proteins is ensured by coupling with a sufficient quantity of β2GPI proteins, either passively in a suspension medium or using a known chemical binding protocol;
b) in a container, said microbeads loaded with β2GPI proteins are brought into contact with the fluid medium M by adding ions of at least one oxidizing metal, under appropriate conditions in order to ensure sufficient binding of the infectious compounds to the β2GPI proteins carried by the microbeads;
c) the microbeads thus prepared are separated from their suspension medium, said suspension medium is removed from the container in order to obtain, optionally after washing with a buffer, a residue with a high concentration of infectious compounds;
d) and the infectious compounds are detected and/or quantified and/or identified, starting from the concentrated residue thus obtained.

2. Method according to claim 1, **characterized by** the fact that the loading of the microbeads, in step a) of claim 1, is carried out passively in a buffer having a pH comprised between 3.5 and 10.5, by incubating, for a period of time comprised between 10 minutes and 24 hours, at a temperature comprised between 4 and 40°C.

3. Method according to one of claims 1 or 2, **characterized by** the fact that the loading of the microbeads with β2GPI proteins is carried out by placing them in a liquid suspension medium which contains, in aqueous solution, from 10⁻⁶ to 100 mg of β2GPI per gram of dry weight of microbeads, the concentration of β2GPI in the medium being comprised between 10⁻⁵ and 10 µg/µl.

4. Method according to one of claims I to 3, **characterized by** the fact that the loading of the microbeads with β2GP1 proteins is carried out in a buffer having a pH comprised between 5 and 9 with incubation for a period of time comprised between 10 minutes and 24 hours at a temperature comprised between 4 and 40°C.

5. Method according to one of claims 1 to 4, **characterized by** the fact that in step b), the ions of oxidizing metal added to the medium M are cupric ions, the concentration of cupric ions in the medium M being comprised, after said addition, between 1 mM and 100 mM.

6. Method according to one of claims 1 to 5, **characterized by** the fact that the solid material constituting the outer surface of the microbeads is chosen from the group formed by the plastics and the elastomers, said material carrying or not carrying reactive groups grafted to the outer surface of the microbeads in order to ensure a chemical bond to the β2GPI proteins.

7. Method according to one of claims 1 to 6, **characterized by** the fact that microbeads having a substantially spherical shape and an average diameter comprised between 1 and 100,000 nm are chosen.

8. Method according to one of claims 1 to 7, **characterized by** the fact that microbeads having a core formed by one (or more) particle(s) of magnetic material are chosen in order to allow their separation from the suspension medium using a magnetic field.

9. Method according to one of claims 1 to 7, **characterized by** the fact that the microbeads are separated from their suspension medium by centrifugation.

10. Method according to one of claims 1 to 9, **characterized by** the fact that step d) of claim 1 is carried out by a means taken from the group formed by infectivity, a specific enzymatic reaction, a fluorescent or radiolabelled tracer, a detection of specific nucleic acids by hybridization with a labelled probe, a PCR or RT-PCR reaction, an assay, a count, a visualization, an optical method, electron or non-electron microscopy.

11. Method according to one of claims 1 to 10, **characterized by** the fact that the infectious compounds are bacteria.

12. Method according to one of claims 1 to 10, **characterized by** the fact that the infectious compounds are viruses.

13. Method according to claim 11, **characterized by** the fact that the bacteria of the fluid medium M are detected and/or quantified and/or identified starting from the residue by optical density reading, by ATP-metry or by PCR.

14. Method according to one of claims 11 to 13, **characterized by** the fact that starting from the concentrated residue, the nucleic acids of the ICs of interest are extracted by lysis, followed by PCR or RT-PCR amplification of said nucleic acids by using the primers appropriate to said ICs of interest and visualization on gel of the nucleic acids optionally obtained in order to define the presence or the absence of said ICs of interest and/or in order to quantify the viral load of said ICs of interest in the medium M.

15. Method according to claim 12, **characterized by** the fact that in order to detect and/or identify viruses of interest bound to microbeads obtained according to step c) of claim 1, the residue is resuspended, these microbeads are brought into contact with cells sensitive to the viruses of interest, said cells are cultured and any infection of the cells by the viruses of interest is observed.

16. Method according to claim 15, **characterized by** the fact that, in order to detect an infection of cells, the cytopathological effect of the viruses after appropriate staining of the cells, or the immunofluorescence after binding of the cells and reaction with fluorescent antibodies which recognize proteins corresponding to the presence of the viruses are observed.

## Patentansprüche

1. Verfahren zum Nachweisen und/oder Quantifizieren und/oder Identifizieren, in vitro, von infektiösen Verbindungen (IV) in einem ein biologisches Material bildenden flüssigen Medium M, wobei bei dem Verfahren auf bekannte Weise in einem flüssigen Suspensionsmedium eine Suspension von Trägerkügelchen hergestellt wird, die von einer Außenfläche begrenzt werden, die von einem festen Polymermaterial gebildet wird, das Proteine fixieren kann, **dadurch gekennzeichnet, dass** es die folgenden Schritte beinhaltet:
a) Beladen der Trägerkügelchen der Suspension mit β2GPI-Proteinen durch Kopplung mit einer ausreichenden Menge an β2GPI-Proteinen, entweder auf passive Weise in einem Suspensionsmedium oder mittels eines bekannten chemischen Verbindungsschemas;
b) Inkontaktbringen, in einem Behälter, der genannten mit β2GPI-Proteinen beladenen Trägerkügelchen mit dem flüssigen Medium M unter Zugabe von Ionen von wenigstens einem oxidierenden Metall, unter geeigneten Bedingungen, um eine ausreichende Fixierung von infektiösen Verbindungen an den von den Trägerkügelchen getragenen β2GPI-Proteinen zu erzielen;
c) Trennen der so hergestellten Trägerkügelchen von ihrem Suspensionsmedium, Evakuieren des genannten Suspensionsmediums aus dem Behälter, um, eventuell nach einer Wäsche in einem Puffer, einen Rest mit hoher Konzentration der infektiösen Verbindungen zu erhalten; und
d) Nachweisen und/oder Quantifizieren und/oder Identifizieren der infektiösen Verbindungen des so erhaltenen konzentrierten Rests.

2. Verfahren nach Anspruch 1, **dadurch gekennzeichnet, dass** die Beladung der Trägerkügelchen in Schritt a) von Anspruch 1 auf passive Weise in einem Puffer mit einem pH-Wert zwischen 3,5 und 10,5 erfolgt, indem sie für eine Zeit zwischen 10 Minuten und 24 Stunden bei einer Temperatur zwischen 4 und 40°C inkubieren gelassen werden.

3. Verfahren nach Anspruch 1 oder 2, **dadurch gekennzeichnet, dass** die Beladung der Trägerkügelchen mit β2GPI-Proteinen erfolgt, indem sie in ein flüssiges Suspensionsmedium gegeben werden, das in wässriger Lösung 10⁻⁶ bis 100 mg β2GPI pro Gramm Trockengewicht Trägerkügelchen enthält, wobei die Konzentration des β2GPI-Mediums zwischen 10⁻⁵ und 10 µg/µl liegt.

4. Verfahren nach einem der Ansprüche 1 bis 3, **dadurch gekennzeichnet, dass** die Beladung der Trägerkügelchen mit β2GPI-Proteinen in einem Puffer mit einem pH-Wert zwischen 5 und 9 mit einer Inkubation für eine Zeit zwischen 10 Minuten und 24 Stunden bei einer Temperatur zwischen 4 und 40°C erfolgt.

5. Verfahren nach einem der Ansprüche 1 bis 4, **dadurch gekennzeichnet, dass** in Schritt b) die dem Medium M zugegebenen Ionen von oxidierendem Metall kupferhaltige Ionen sind, wobei die Konzentration der kupferhaltigen Ionen in dem Medium M nach der genannten Zugabe zwischen 1 mM und 100 mM liegt.

6. Verfahren nach einem der Ansprüche 1 bis 5, **dadurch gekennzeichnet, dass** das die Außenfläche der Trägerkügelchen bildende feste Material ausgewählt wird aus der Gruppe bestehend aus Kunststoffen und Elastomeren, wobei das genannte Material reaktionsfähige Gruppen trägt oder nicht, die auf die Außenfläche der Trägerkügelchen aufgepropft sind, um eine chemische Verbindung mit den β2GPI-Proteinen zu bewirken.

7. Verfahren nach einem der Ansprüche 1 bis 6, **dadurch gekennzeichnet, dass** Trägerkügelchen mit einer im Wesentlichen sphärischen Form und einem mittleren Durchmesser zwischen 1 und 100.000 nm gewählt werden.

8. Verfahren nach einem der Ansprüche 1 bis 7, **dadurch gekennzeichnet, dass** Trägerkügelchen mit einem Kern gewählt werden, der aus einem oder mehreren Partikeln aus magnetischem Material gebildet ist, um deren Trennung in Bezug auf das Suspensionsmedium mittels eines magnetischen Feldes zuzulassen.

9. Verfahren nach einem der Ansprüche 1 bis 7, **dadurch gekennzeichnet, dass** die Trägerkügelchen durch Zentrifugieren von ihrem Suspensionsmedium getrennt werden.

10. Verfahren nach einem der Ansprüche 1 bis 9, **dadurch gekennzeichnet, dass** Schritt d) von Anspruch 1 mit einem Mittel durchgeführt wird, das ausgewählt wird aus der Gruppe bestehend aus Infektiosität, spezifische Enzymreaktion, fluoreszierendem oder radiomarkiertem Indikator, Nachweis von spezifischen Nukleinsäuren durch Hybridisierung mit einer markierten Sonde, einer PCR- oder RT-PCR-Reaktion, Dosierung, Nummerierung, Visualisierung, einem optischem Verfahren, einem Elektronenmikroskop oder nicht.

11. Verfahren nach einem der Ansprüche 1 bis 10, **dadurch gekennzeichnet, dass** die infektiösen Verbindungen Bakterien sind.

12. Verfahren nach einem der Ansprüche 1 bis 10, **dadurch gekennzeichnet, dass** die infektiösen Verbindungen Viren sind.

13. Verfahren nach Anspruch 11, **dadurch gekennzeichnet, dass** die Bakterien des flüssigen Mediums M vom Rest durch Lesen der optischen Dichte, durch ATP-Metrik oder durch PCR nachgewiesen und/oder quantifiziert und/oder identifiziert werden.

14. Verfahren nach einem der Ansprüche 11 bis 13, **dadurch gekennzeichnet, dass** die Nukleinsäuren der CI von Interesse durch Lyse vom konzentrierten Rest extrahiert werden, dass die genannten Nukleinsäuren durch PCR oder RT-PCR unter Verwendung von geeigneten Primern an den genannten CI von Interesse amplifiziert werden und dass eine Visualisierung auf Gel von eventuell erhaltenen Nukleinsäuren durchgeführt wird, um die An- oder Abwesenheit der genannten CI von Interesse zu definieren und/oder um die Virusbeladung der genannten CI von Interesse in dem Medium M zu quantifizieren.

15. Verfahren nach Anspruch 12, **dadurch gekennzeichnet, dass** zum Nachweisen und/oder Identifizieren der auf den in Schritt c) von Anspruch 1 erhaltenen Trägerkügelchen fixierten Viren von Interesse der Rest resuspendiert, diese Trägerkügelchen mit den für die Viren von Interesse empfindlichen Zellen in Kontakt gebracht und die genannten Zellen in Kultur gebracht und eine eventuelle Infektion der Zellen durch die Viren von Interesse beobachtet wird.

16. Verfahren nach Anspruch 15, **dadurch gekennzeichnet, dass** zum Nachweisen einer Zelleninfektion der zytopathologische Effekt der Viren nach einer geeigneten Färbung der Zellen oder die Immunofluoreszenz nach der Fixierung der Zellen und der Reaktion mit den fluoreszierenden Antikörpern beobachtet wird, die die Proteine entsprechend der Anwesenheit der Viren erkennen.
